# EUROPÄISCHE PATENTSCHRIFT

(11) **EP 1 411 842 B1**
(45) Veröffentlichungstag und Bekanntmachung des Hinweises auf die Patenterteilung: **23.09.2009**
(21) Anmeldenummer: 02758320.2
(22) Anmeldetag: 06.07.2002
(51) Int. Cl.: A61B 17/28, A61B 17/30, A61B 17/32

(54) **CHIRURGISCHES INSTRUMENT ZUM KLEMMEN UND SCHNEIDEN**
SURGICAL INSTRUMENT FOR CLAMPING AND CUTTING
INSTRUMENT CHIRURGICAL SERVANT A PINCER ET A COUPER

(30) Priorität: 30.07.2001 DE 10137124
(43) Veröffentlichungstag der Anmeldung: 28.04.2004
(73) Patentinhaber: Aesculap AG, 78532 Tuttlingen (DE)
(72) Erfinder: DWORSCHAK, Manfred, 78589 Dürbheim (DE); LUTZE, Theodor, 78582 Balgheim (DE); MORALES, Pedro, 78532 Tuttlingen-Nendingen (DE); WEISSHAUPT, Dieter, 78194 Immendingen (DE)
(74) Vertreter: Regelmann, Thomas
(86) Internationale Anmeldenummer: PCT/EP2002/007562
(87) Internationale Veröffentlichungsnummer: WO 2003/011152

(56) Entgegenhaltungen:
- DE-A- 3 322 741
- DE-U- 20 001 204
- US-A- 6 146 399

## Beschreibung

Die Erfindung betrifft ein chirurgisches Instrument mit einem Schenkel, welcher an einen Gegenstand anlegbar ist.

Bei einem solchen chirurgisches Instrument kann es sich beispielsweise um einen Tupfer handeln oder um eine Zange, welche beispielsweise als Pinzette oder als Klemme ausgebildet sein kann.

Ein bipolares Faßinstrument, das die Merkmale des Oberbegriffs des Anspruchs 1 besitzt, ist aus der Gebrauchsmusterschrift DE 200 01 204 U1 bekannt.

Der Erfindung liegt die Aufgabe zugrunde, ein chirurgisches Instrument zu schaffen, welches auf vielfältige Weise mit leichter Steuerbarkeit seiner Funktionsfähigkeiten einsetzbar ist.

Diese Aufgabe wird bei einem chirurgischen Instrument der eingangs genannten Art erfindungsgemäß dadurch gelöst, daß an dem Schenkel ein Messerlager mit einem Messer angeordnet ist, daß das Messerlager eine Anlagefläche für den Gegenstand aufweist und daß das Messerlager so ausgebildet ist, daß in einer Nichtwirkstellung eine Schneidkante des Messers unterhalb der Anlagefläche liegt und in einer Wirkstellung des Messers die Schneidkante freigegeben ist, wobei in dem Messerlager ein Polster aus einem elastischen Material angeordnet, welches mindestens in der Nichtwirkstellung die Anlagefläche für den Gegenstand bildet. Das Polster ist dabei so zusammendrückbar, daß die Schneidkante des Messers freigegeben ist. Entsprechend läßt sich durch Zusammendrücken des Polsters die relative Lage zwischen Schneidkante und Anlagefläche ändern, wobei dieses Zusammendrücken wiederum durch Kraftbeaufschlagung des Schenkels relativ zu dem Gegenstand erreicht ist. Durch ein solches "gebettetes" Messer läßt sich ein Umschalten zwischen der Funktion des Fassens (leichtes Schließen) und der Funktion des Schneidens (festes Schließen) erreichen.

Mit einem solchen chirurgischen Instrument läßt sich über den Übergang zwischen der Nichtwirkstellung und der Wirkstellung eine Schneidbearbeitung des Gegenstandes durchführen. In der Nichtwirkstellung läßt sich dabei der Gegenstand ohne Schneidbearbeitung behandeln; bei einer Klemme oder einer Pinzette läßt sich beispielsweise der entsprechende Gegenstand fassen.

Günstigerweise ist dabei ein weiterer Schenkel vorgesehen, wobei die beiden Schenkel relativ zueinander beweglich sind. Dadurch läßt sich dann der Gegenstand zwischen den beiden Schenkeln fassen und auch für eine Schneidbewegung ist ein Gegenlager zum Halten des Gegenstandes bereits vorgegeben. Dadurch läßt sich auf einfache Weise ein zwischen den beiden Schenkeln gehaltener Gegenstand während des Haltens schneidbearbeiten.

Vorteilhafterweise ist dazu die Anlagefläche des Messerlagers dem weiteren Schenkel zugewandt angeordnet, um so den Gegenstand sicher fassen zu können.

Weiterhin ist es zum sicheren Halten günstig, wenn der weitere Schenkel ein Gegenlager mit einer Anlagefläche für den Gegenstand aufweist. Insbesondere kann es dabei vorgesehen sein, daß das Messerlager und das Gegenlager bezüglich ihrer Anlageflächen im wesentlichen gleich ausgebildet sind, um so ein symmetrisches Fassen und Halten des Gegenstandes zu gewährleisten, bei dem Kraftspitzen auf den Gegenstand minimiert sind.

Gans besonders vorteilhaft ist es, wenn der Übergang der Schneidkante von der Nichtwirkstellung in die Wirkstellung durch Kraftbeaufschlagung des Schenkels relativ zum Gegenstand steuerbar ist und insbesondere durch Kraftbeaufschlagung der beiden Schenkel, wenn zwei Schenkel vorgesehen sind, relativ zueinander steuerbar ist.

Dadurch kann ein Bediener, beispielsweise ein Operateur, durch Aufeinanderbewegung der beiden Schenkel gesteuert die Funktion des Fassens und den Übergang zum Schneiden einstellen.

Um einen Gegenstand schneidbearbeiten zu können, ist in der Wirkstellung des Messers die Schneidkante oberhalb der Anlagefläche positioniert. Der Übergang zwischen Nichtwirkstellung und Wirkstellung erfolgt dann also durch Änderung der relativen Lage zwischen der Schneidkante und der Anlagefläche. Insbesondere ist dabei diese relative Lage steuerbar, um so den Funktionsübergang steuern zu können. Diese Steuerung läßt sich wiederum auf einfache Weise durchführen, wenn die relative Lage zwischen Anlagefläche und Schneidkante durch Kraftbeaufschlagung des Schenkels steuerbar ist, die auf einfache Weise vorgegeben werden kann.

Bei einer Variante einer Ausführungsform ist das Messer zwischen einem ersten Polsterteil und einem zweiten Polsterteil angeordnet. Das Messer kann dabei fest an dem Schenkel sitzen oder auch verschieblich in diesem geführt sein.

Ferner ist es günstig, wenn in einer Wirkstellung des Messers die Anlagefläche zumindest teilweise durch eine Polsteraufnahme des Messerlagers gebildet ist und dabei insbesondere durch den Gegenstand hin orientierte Stirnseiten von Wänden der Polsteraufnahme gebildet ist. Dadurch ist gewährleistet, daß bei genügender Kraftbeaufschlagung der Gegenstand stets an dem Messerlager anliegt und dann die Schneidkante oberhalb des Messerlagers ist, so daß damit auch stets gewährleistet ist, daß der Gegenstand schneidbearbeitbar ist.

Das Gegenlager kann dabei ebenfalls mit einem Polster aus einem elastischen Material versehen sein, um auch so ein sicheres Fassen zu ermöglichen. Es ist dadurch auch erreichbar, daß das Messer in das Polster des Gegenlagers einschneiden kann, wenn die Kraftbeaufschlagung des Gegenstandes über das Messerlagers nicht gleichmäßig ist; dadurch läßt sich der entsprechende Gegenstand eben auch dann schneidbearbeiten, wenn beispielsweise die beiden Schenkel aufgrund des Gegenstandes dazwischen nicht mehr exakt schließen können.

Bei einer Variante einer Ausführungsform ist vorgesehen, daß das Messer zur Durchführung einer Schneidbewegung an dem Schenkel beweglich gelagert ist. Dadurch läßt sich beispielsweise ein ziehender Schnitt in einen Gegenstand, welcher zwischen zwei Schenkeln gefaßt ist, einbringen.

Insbesondere ist dazu das Messer in einer Längsrichtung des Schenkels verschieblich gelagert, so daß die Beweglichkeit des Messers die Steuerung des Übergangs zwischen der Nichtwirkstellung in die Wirkstellung im wesentlichen unbeeinflußt läßt.

Zur einfachen Durchführung der Verschiebung weist das Messer günstigerweise ein Griffelement zu dessen Betätigung auf. Es kann sich dabei insbesondere um eine Griffmulde handeln, die so an dem Schenkel angeordnet ist, daß das Messer relativ zu diesem verschieblich führbar ist.

Weiterhin ist es günstig, wenn eine Sicherungsvorrichtung gegen eine unbeabsichtigte Schneidbewegung des Messers vorgesehen ist, das heißt die eine Beweglichkeit des Messers an dem Schenkel sperrt, sofern die Sicherungsvorrichtung die Verschiebung nicht freigibt.

Bei einer Variante einer Ausführungsform ist die Schneidkante quer zur Anlagefläche orientiert. Beispielsweise kann das Messer ein hakenförmiges Element aufweisen, in welchem die Schneidkante sitzt. Auf diese Weise läßt sich ein ziehender Schnitt in einen Gegenstand einbringen.

Es kann alternativ vorgesehen sein, daß die Schneidkante des Messers im wesentlichen parallel zu der Anlagefläche orientiert ist, um so beispielsweise einen Gegenstand wie einen Faden durch die Aufeinanderbewegung der Schenkel durchtrennen zu können.

Günstigerweise ist, sofern das Messer beweglich ausgebildet ist, ein Anschlag zur Begrenzung der Beweglichkeit vorgesehen. Dadurch sind insbesondere eine Ausgangslage und eine Endstellung des Messers genau definiert, so daß diese ausgezeichneten Stellungen sich definiert erreichen lassen.

Insbesondere ist ein Anschlag in einem vorderen Ende des Schenkels angeordnet, um ein Messer in die Ausgangsstellung führen zu können. Durch das Erreichen dieses Anschlages läßt sich dann auch eine Sicherungsstellung erreichen, in der eine Sicherungsvorrichtung die Beweglichkeit sperrt.

Bei einer Variante einer Ausführungsform ist das Messer und eine Messerführung so ausgebildet, daß bei Verschiebung des Messers sich die Lage der Schneidkante relativ zur Anlagefläche ändert. Dadurch läßt es sich beispielsweise sicherstellen, daß ein Schnitt in einen entsprechenden Gegenstand eingebracht wird, da sich durch die Messerverschiebung bereits die relative Lage zwischen Schneidkante und Anlagefläche ändert.

Gans besonders vorteilhaft ist es, wenn eine Begrenzungsvorrichtung vorgesehen ist, mittels welcher die Relativbewegung der beiden Schenkel zueinander derart begrenzbar ist, daß das Messer in der Nichtwirkstellung bleibt. Die Begrenzungsvorrichtung sichert dann die Funktion des Fassens, ohne daß die Gefahr besteht, daß eine Schneidbearbeitung des Gegenstandes durchgeführt wird.

Insbesondere ist durch die Begrenzungsvorrichtung der Anpreßdruck des Schenkels mit dem Messerlager auf einen Gegenstand an der Anlagefläche begrenzbar, um so ein erfindungsgemäßes chirurgisches Instrument in seiner Haltefunktion zu sichern.

Günstigerweise ist die Begrenzungsvorrichtung zwischen mindestens einer Begrenzungsstellung und einer Nichtbegrenzungsstellung umschaltbar, wobei dann in letzterer ein Gegenstand, welcher zwischen den beiden Schenkeln gehalten ist, sich schneidbearbeiten läßt.

Bei einem ersten Ausführungsbeispiel ist das chirurgische Instrument als Zange bzw. Pinzette ausgebildet, bei der der Schenkel mit einem weiteren Schenkel derart elastisch verbunden ist, daß die beiden Schenkel relativ zueinander beweglich sind. Mittels einer solchen Zange bzw. Pinzette läßt sich dann ein Gegenstand aber nicht zur halten, sondern auch schneidbearbeiten. Beispielsweise läßt sich dadurch ein Faden durchtrennen oder es lassen sich während einer Operation gehaltene Gegenstände auch durchschneiden.

Bei einem zweiten Ausführungsbeispiel ist ein anderer Schenkel vorgesehen, welcher drehbar an dem Schenkel angeordnet ist. Ein solches chirurgisches Instrument ist insbesondere als Klemme ausgebildet.

Es läßt sich günstigerweise erreichen, daß der Schenkel mit dem Messerlager so ausgebildet ist, daß bei Schließen der Klemme die Anlagefläche an einer Anlagefläche des anderen Schenkels im wesentlichen anliegt. Dadurch läßt sich auf sichere Weise ein Gegenstand zwischen den beiden Schenkeln halten.

Ganz besonders vorteilhaft ist es, wenn der Schenkel so elastisch ausgebildet ist, daß das Messerlager relativ zu einer Längsrichtung des Schenkels beweglich ist. Das Messerlager ist dabei insbesondere beim Anliegen an den anderen Schenkel und bei Kraftausübung der beiden Schenkel relativ zueinander von dem anderen Schenkel weg beweglich. Auf diese Weise läßt sich bei Kraftausübung ein Heraustreten der Schneidkante aus dem Messerlager über die Anlagefläche heraus erreichen, um so beispielsweise einen zwischen den Schenkeln gehaltenen Faden zu durchtrennen.

Eine solche Ausbildung läßt sich bei entsprechender Materialwahl erreichen, wenn der Schenkel an seiner dem anderen Schenkel abgewandten Seite eine Ausnehmung aufweist. Diese Ausnehmung stellt eine Materialverdünnung im Schenkel dar und dadurch läßt sich bei Kraftausübung das elastische Ausweichen des entsprechenden Schenkelteils und damit des Messerlagers steuern.

Ganz besonders vorteilhaft ist es, wenn ein Schenkel aus einem Kunststoffmaterial hergestellt ist. Ein solches chirurgisches Instrument läßt sich dadurch einfach und kostengünstig herstellen.

Die nachfolgende Beschreibung bevorzugter Ausführungsformen dient im Zusammenhang mit der Zeichnung der näheren Erläuterung der Erfindung. Es zeigen:
- Figur 1: ein erstes Ausführungsbeispiel einer erfindungsgemäßen Pinzette;
- Figur 2: eine Schnittansicht längs der Linie 2-2 gemäß Figur 1, wobei ein Messer in einer Nichtwirkstellung ist;
- Figur 3: eine Längsschnittansicht auf einen Schenkel mit einem Messerlager, und einem ersten Ausführungsbeispiel eines Messers;
- Figur 4: eine ähnliche Ansicht wie Figur 3 aber mit einem zweiten Ausführungsbeispiel eines Messers;
- Figur 5: eine Schnittansicht eines Schenkels mit einer Sicherungsvorrichtung gegen unbeabsichtigte Schneidbewegung eines Messers und
- Figur 6: eine Ausführungsform einer erfindungsgemäßen Klemme.

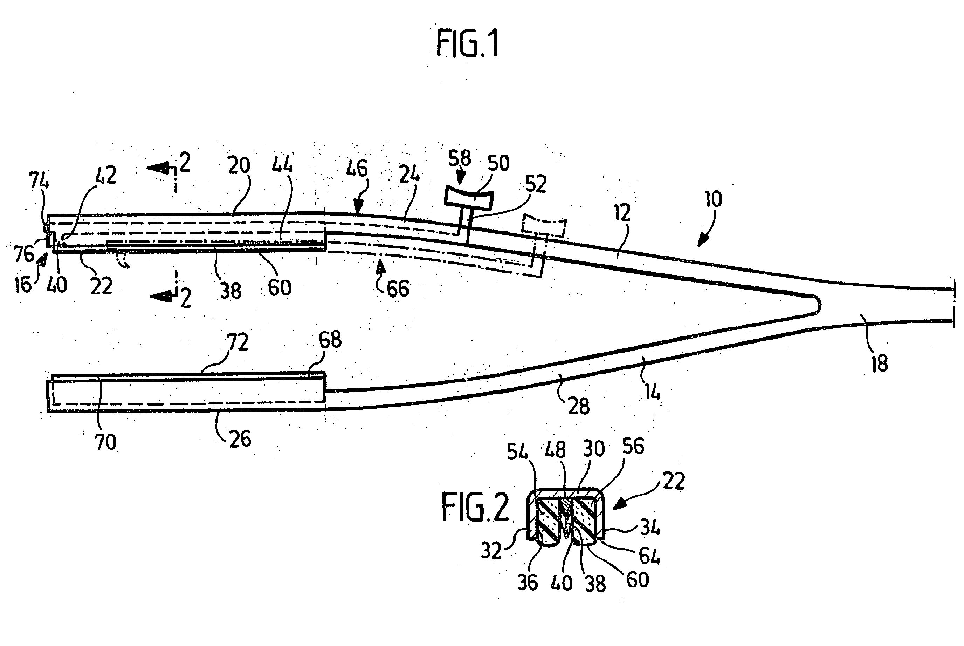

Ein erfindungsgemäßes chirurgisches Instrument kann beispielsweise als Zange und insbesondere als Pinzette oder als Klemme ausgebildet sein. In Figur 1 ist eine Pinzette dargestellt, welche als Ganzes mit 10 bezeichnet ist. Diese Pinzette weist einen ersten Schenkel 12 und einen zweiten Schenkel 14 auf, welche an dem ihren jeweiligen Wirkende 16 gegenüberliegenden Ende miteinander über ein Verbindungsteil 18 verbunden sind. Dadurch weist die Pinzette 10 eine V-förmige Gestalt auf.

Die beiden Schenkel 12 und 14 sind relativ zueinander beweglich, so daß zwischen ihnen ein Gegenstand faßbar ist. Sie sind dazu aus einem elastischen Material gefertigt, so daß ein Schenkel 12, 14 relativ zu dem Verbindungsteil 18 beweglich ist.

Der erste Schenkel 12 weist dabei ein Vorderteil 20 auf, an dem ein Messerlager 22 angeordnet ist. Einstückig mit diesem Vorderteil 20 verbunden ist ein Mittelteil 24, welches in einem Winkel zu der Längsrichtung des Vorderteils 20 angeordnet ist. Dieses Mittelteil 20 wiederum ist einstückig mit dem Verbindungsteil 18 verbunden.

Der zweite Schenkel 14 weist ebenfalls ein Vorderteil 26 und ein einstückig mit diesem verbundenes Mittelteil 28 auf, welches wiederum einstückig an dem Verbindungsteil 18 sitzt. Dadurch ist die Pinzette 10 im wesentlichen zu einer Mittelebene durch das Verbindungsteil 18 symmetrisch hinsichtlich der Ausbildung der Schenkel 12 und 14 ausgestaltet.

Die Schenkel 12 und 14 und das Verbindungsteil 18 sind dabei insbesondere aus einem Kunststoffmaterial wie LCP oder PPA hergestellt.

Im Bereich seines Vorderteils 20 ist der erste Schenkel 12 wannenförmig ausgebildet (Figur 2), um das Messerlager 22 zu bilden. Beispielsweise weist das Vorderteil 20 dazu einen Schenkelboden 30 auf, an welchem im wesentlichen senkrecht beabstandete Schenkelwände 32 und 34 sitzen, so daß zwischen dem Schenkelboden 30 und den Schenkelwänden 32 und 34 eine im Querschnitt im wesentlichen rechteckförmige Aufnahme 36 gebildet ist.

In dieser Aufnahme 36 sitzt ein Messer 38, welches insbesondere aus einem metallischen Material hergestellt ist. Dieses Messer 38 sitzt dabei vorzugsweise im wesentlichen mittig zwischen den Schenkelwänden 32 und 34. Bei dem in den Figuren 1 und 2 gezeigten Ausführungsbeispiel weist das Messer ein hakenförmiges Element 40 auf, an dem eine Schneidkante 42 gebildet ist, welche quer zu dem Schenkelboden 30 orientiert ist.

Das hakenförmige Element 40 sitzt dabei an einem Messerteil 44 beispielsweise einem Blechteil, und insbesondere einstückig an diesem. Das Messerteil 44 ist relativ zu dem ersten Schenkel 12 in dessen Längsrichtung verschieblich über eine als Ganzes mit 46 bezeichnete Verschiebeführung geführt. Beispielsweise umfaßt die Verschiebeführung 46 eine Führungsleiste 48, welche an dem Schenkelboden 30 angeordnet ist und in welcher das Messerteil 44 mit dem hakenförmigen Element 40 geführt ist.

Das Messerteil 44 wiederum ist mit einem Betätigungselement 50 verbunden, welches beispielsweise in der Form einer Griffmulde ausgebildet ist, und das über den Schenkelboden 30 in Richtung der dem Messer 38 abgewandten Seite hinaussteht. Die Griffmulde 50 ist dabei über ein Zwischenteil 52 mit dem Messerteil 44 verbunden, wobei das Zwischenteil 52 in dem entsprechenden Teilbereich des Schenkelbodens 30 beispielsweise über einen Führungsschlitz geführt ist. Über die Griffmulde 50 läßt sich dann das Messer 38 mit der Schneidkante 42 längs des ersten Schenkels 12 verschieben. Insbesondere läßt sich dadurch eine Schneidbewegung so durchführen, daß über die Griffmulde 50 das Messer 38 von dem Wirkende 16 weg in Richtung des Verbindungsteils 18 verschoben wird.

Wie in Figur 2 gezeigt, ist zwischen dem Messer 38 und den Schenkelwänden 32, 34 jeweils ein Polster 54, 56 aus einem elastischen Material wie beispielsweise Silikon angeordnet. In einer Nichtwirkstellung 58, welche in Figur 1 in durchgezogenen Linien gezeichnet ist und ebenfalls in Figur 2 gezeigt ist, weist der erste Schenkel 12 eine Anlagefläche 60 für einen Gegenstand auf, welche durch eine dem anderen Schenkel 14 zugewandte Oberfläche der Polster 54, 56 gebildet ist. Das Messer 38 mit seiner Schneidkante 42 liegt in dieser Nichtwirkstellung 58 unterhalb der Anlagefläche 60, so daß ein an der Anlagefläche 60 anliegender Gegenstand nicht mit dem Messer in Berührung kommt.

Wird nun auf die Polster 54, 56 ein Druck ausgeübt, so bewirkt die Elastizität des Materials, daß sich die relative Lage zwischen der Schneidkante 42 und einer die Anlagefläche 60 bildenden Oberfläche der Polster 54, 56 ändert. Ist der auf die Polster 54, 56 ausgeübte Druck genügend groß, so wird die Anlagefläche für den Gegenstand schließlich durch eine Stirnseite 62 der Schenkelwand 32 und eine Stirnseite 64 der Schenkelwand 34 gebildet und die Schneidkante 42 ragt über die Anlagefläche 60 hinaus, so daß sie auf den anliegenden Gegenstand einwirken kann.

In Figur 1 ist in strichpunktierten Linien eine solche Wirkstellung 66 gezeigt, in welcher die Schneidkante 42 des Messers 38 über die Anlagefläche 60 hinausragt und damit auf einen Gegenstand wirken kann.

Der zweite Schenkel 14 weist ein Gegenlager 68 auf, welches grundsätzlich gleich ausgebildet ist wie das Messerlager 22, ohne daß ein Messer vorgesehen ist. In diesem Gegenlager 68 ist ebenfalls ein Polster 70 aus einem elastischen Material angeordnet, durch welches eine Anlagefläche 72 dem anderen Schenkel 12 zugewandt gebildet ist. Das Polster 70 ist dabei vorzugsweise einstückig in einer entsprechenden Aufnahme angeordnet, welche grundsätzlich gleich ausgebildet ist wie die Aufnahme 36 des Messerlagers 22.

Die erfindungsgemäße Pinzette funktioniert nun wie folgt:

Zum Fassen eines Gegenstandes wird dieser zwischen den ersten Schenkel 12 und dem zweiten Schenkel 14 der Pinzette 10 gebracht und insbesondere zwischen das Messerlager 22 und das Gegenlager 68. Durch Relativbewegung der Schenkel 12 und 14 zueinander wird der Gegenstand gefaßt, wobei er dann an den Anlageflächen 60 des ersten Schenkels 12 und der Anlagefläche 72 des zweiten Schenkels 14 anliegt. Ist dabei die Kraftbeaufschlagung der Schenkel 12 und 14 zu dem Gegenstand gerade so, daß dieser gerade gefaßt wird, dann liegt das Messer 38 in seiner Nichtwirkstellung 58, das heißt unterhalb der Anlagefläche 60 und kann damit auch nicht auf den Gegenstand einwirken.

Wird jedoch die Kraftbeaufschlagung verstärkt, das heißt werden die Schenkel 12 und 14 stärker zusammengedrückt, dann werden entsprechend das Polster 70 und die Polster 54 und 56 zusammengedrückt. Durch festeres Schließen der Pinzette 10 läßt es sich dann bewirken, daß die Schneidkante 42 über die Anlagefläche 60 hinaustritt. Im Extremfall ist dann die Anlagefläche 60 am Messerlager 22 durch die Stirnseiten 62, 64 der Schenkelwände 32, 34 gebildet und entsprechend beim Gegenlager 68 durch die Stirnseiten der zugeordneten Schenkelwände.

In der Wirkstellung 66 kann dann der Gegenstand schneidbearbeitet werden, indem über die Griffmulde 50 das Messer 38 in Richtung des Verbindungsteils 18 gezogen wird; das Messer 38 mit der Schneidkante 42 führt dann einen ziehenden Schnitt durch.

Erfindungsgemäß werden also die Funktionen Fassen eines Gegenstandes und Schneidbearbeitung eines Gegenstandes über die Kraftbeaufschlagung der beiden Schenkel 12, 14 relativ zu dem Gegenstand gesteuert; durch leichtes Schließen läßt sich der Gegenstand fassen, wobei dort die Schneidkante 42 versenkt ist und durch entsprechendes festes Schließen läßt sich der gefaßte Gegenstand schneidbearbeiten, indem die Schneidkante 42 über die Anlagefläche 60 hinaustritt. Bei dem in Figur 1 gezeigten Ausführungsbeispiel wird dann die Schneidbewegung selber durch Verschiebung des Messers 38 in Richtung des Verbindungsteils 18 durchgeführt.

Es kann dabei eine Begrenzungsvorrichtung vorgesehen sein, welche die relative Kraftausübung der beiden Schenkel 12, 14 zueinander (das heißt deren relative Beweglichkeit zueinander) derart begrenzt, daß insbesondere sichergestellt ist, daß beim Fassen eines Gegenstandes die Schneidkante 42 nicht über die Anlagefläche hinaustritt; damit läßt sich sicherstellen, daß ein Gegenstand nicht unbeabsichtigt schneidbearbeitet wird. Soll ein gefaßter Gegenstand auch schneidbearbeitet werden, dann muß zuvor die Begrenzungsvorrichtung so eingestellt werden, daß sie einen weiteren Zuwachs der Kraftbeaufschlagung erlaubt, um von der Nichtwirkstellung 58 des Messers 38 in die Wirkstellung 66 übergehen zu können. Eine solche Begrenzungsvorrichtung wird untenstehend noch anhand des Ausführungsbeispiels einer Klemme erläutert.

Zur genauen Definition der Ausgangslage des Messers 38, von der aus eine Ziehbewegung zur Durchführung einer Schneidbewegung durchführbar ist, weist das Messer 38 an einer Stirnseite, welche dem Wirkende 16 des ersten Schenkels 12 zugewandt ist, eine Nase 74 auf, welche als Sicherungsnase dient. Das Wirkende 16 des ersten Schenkels 12 weist dabei eine Ausnehmung 76 auf, in die die Nase 74 eintauchen kann. Ist dabei die Nase 74 in die Ausnehmung 76 eingetaucht, dann ist ein Anschlag für das Messer 38 an einer Innenseite einer Wand gebildet; diese Wand wiederum bildet das Wirkende 16 des ersten Schenkels 12 und schließt damit das Messerlager 22 an dem Wirkende 16 ab. Durch eine solche Ausbildung einer Nase 74 und zugeordnete Ausnehmung im Schenkel 12 läßt sich eine definierte Ausgangsposition für das Messer 38 (und damit auch eine Sicherung gegenüber Herausziehen des Messers 38 in Richtung des Wirkendes 16) herstellen, auch wenn beispielsweise das hakenförmige Element 40 gekrümmt ist.

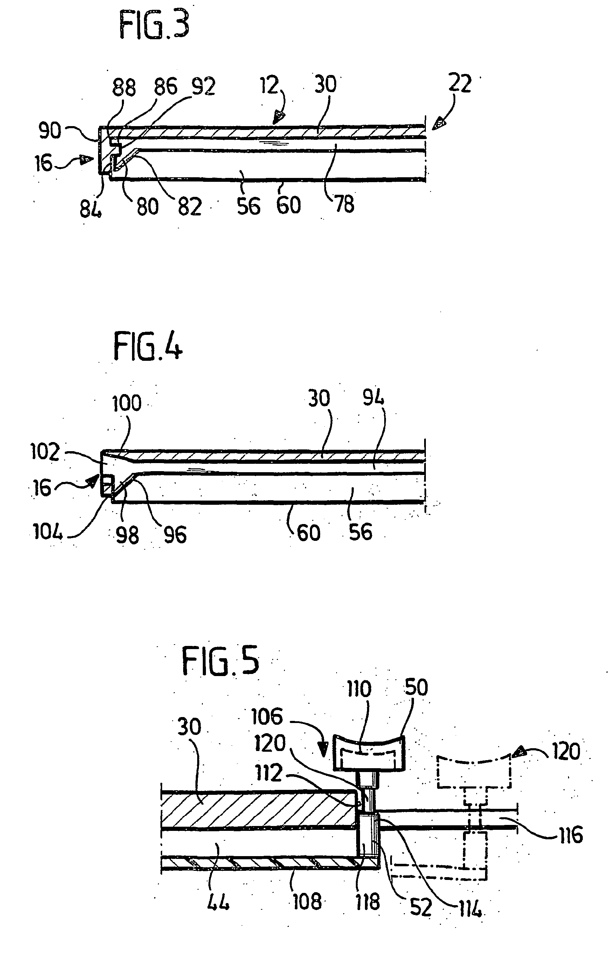

Bei einer Variante einer Ausführungsform, welche in Figur 3 gezeigt ist, ist ein Messer 78 vorgesehen, welches wiederum in dem Messerlager 22 angeordnet ist, welches ein dreieckförmiges Element 80 aufweist, an dem eine Schneidkante 82 gebildet ist, welche in einem Winkel beispielsweise der Größenordnung von 60° zu dem Schenkelboden 30 des Messerlagers 22 orientiert ist.

In einer Stirnkante 84 des Messers 78, welche dem Wirkende 16 zugewandt ist, ist eine Ausnehmung 86 gebildet. Eine Innenwand 88 des ersten Schenkels 12, welche einer Außenwand 90 gegenüber liegt, welche wiederum das vordere Ende 16 bildet, ist eine Nase 92 angeordnet, die mit der Ausnehmung 86 im Messer 78 korrespondiert. Dadurch ist ebenfalls eine Begrenzung für die Verschieblichkeit des Messers 78 in Richtung des Wirkendes 16 hergestellt.

Die Nase 92 muß dabei nicht unbedingt an einer Innenwand 88 angeordnet sein, sondern kann beispielsweise auch durch eine Leiste zwischen den Schenkelwänden 32 und 34 gebildet sein. Auf diese Weise läßt sich die Schneidkante 82 bis ganz zum vorderen Ende des ersten Schenkels 12 verschieben.

Bei einer weiteren Variante eines Ausführungsbeispiels, welche in Figur 4 gezeigt ist, ist ein Messer 94 vorgesehen, welches eine Schneidkante 96 aufweist, die an einem dreieckförmigen Element 98 gebildet ist.

An dem Wirkende 16 ist dabei der Boden 30 mit einer Abfasung 100 versehen, so daß die Dicke des Bodens zu dem Wirkende 16 hin stetig und insbesondere linear abnimmt.

Das Messer 96 wiederum ist mit einem Element und insbesondere dreieckförmigen Element 102 versehen, welches an diese Abfasung 100 angepaßt ist. In der Nichtwirkstellung 58 liegt das Element 102 in der Abfasung 100, wobei eine Kante des dreieckförmigen Elements 98, an welcher die Schneidkante 96 gebildet ist, an einem Anschlag 104 des ersten Schenkels 12 anliegt. Dieser Anschlag 104 ist beispielsweise durch eine Leiste zwischen den Schenkelwänden 32 und 34 gebildet.

Wird nun das Messer 94 in Richtung des Verbindungsteils 18 bewegt, so muß dabei das dreieckförmige Element 102 aus der Fase 100 herausbewegt werden. Dadurch ist eine Zwangsführung für das Element 98 mit der Schneidkante 96 gebildet, über die der Höhenabstand der Schneidkante 96 zu dem Boden 30 vergrößert wird. Dadurch wird beim Zurückziehen die Schneidkante 96 relativ zu dem Polster 56 auch in einer Richtung quer zur Verschiebungsrichtung bewegt, das heißt der Abstand zur Anlagefläche 60 wird verringert.

Durch eine solche Ausbildung des Schenkelbodens 30 und des Messers 94 läßt sich der Übergang zwischen der Nichtwirkstellung 58 und der Wirkstellung 66 zusätzlich steuern und zudem läßt sich die Schneidbewegung des Messers 94 an dem Gegenstand steuern.

Um eine ungewollte Längsverschiebung des Messers 38 bzw. 78, 94 zu verhindern, kann eine Sicherungsvorrichtung vorgesehen sein, welche in Figur 5 als Ganzes mit 106 bezeichnet ist. Das Zwischenteil 52, in welchem die Griffmulde 50 sitzt, ist dabei mit einer Feder 108, beispielsweise einer Kunststofffeder verbunden. Diese Feder 108 wiederum ist mit dem Messerteil 44 verbunden. Unter Druck auf das Griffteil 50 läßt sich die Feder 108 von dem Boden 30 wegdrücken, das heißt die Griffmulde 50 läßt sich in Richtung des Bodens 30 drücken (durch Bezugszeichen 110 in Figur 5 angedeutet).

Ist die Griffmulde 50 nicht kraftbeaufschlagt, so ist der erste Schenkel 12 so ausgebildet, daß das Zwischenteil 52 in seiner Führung 112 quer zur Längsrichtung des Schenkels 12 an einen Anschlag 114 anstößt, welcher damit die Bewegung des Messerteils 44 längs des Schenkels 12 sperrt. In einem entsprechenden Führungsteil 116 des ersten Schenkels 12 ist dabei ein Führungsschlitz angeordnet (in Figur 5 nicht gezeigt). Das Zwischenteil 52 weist dabei einen Bereich 118 mit einem größeren Durchmesser und einen Bereich 120 mit einem kleineren Durchmesser auf. Der Führungsschlitz ist so dimensioniert, daß der Bereich 118 mit größeren Durchmesser nicht in ihn eintauchen kann, das heißt, daß der Anschlag 114 wirkt, während der Bereich 120 in ihn eintauchen kann. Durch Druckausübung auf die Griffmulde 50 in Richtung des Schenkelbodens 30 wird dann der Bereich 120 kleineren Durchmessers mit diesem Führungsschlitz ausgerichtet und der Bereich 120 kann in diesen eintauchen. Dadurch wiederum kann der Bereich 120 in dem Führungsschlitz verschoben werden und damit läßt sich das Messer 38 verschieben. Dies ist in Figur 5 durch das Bezugszeichen 120 angedeutet.

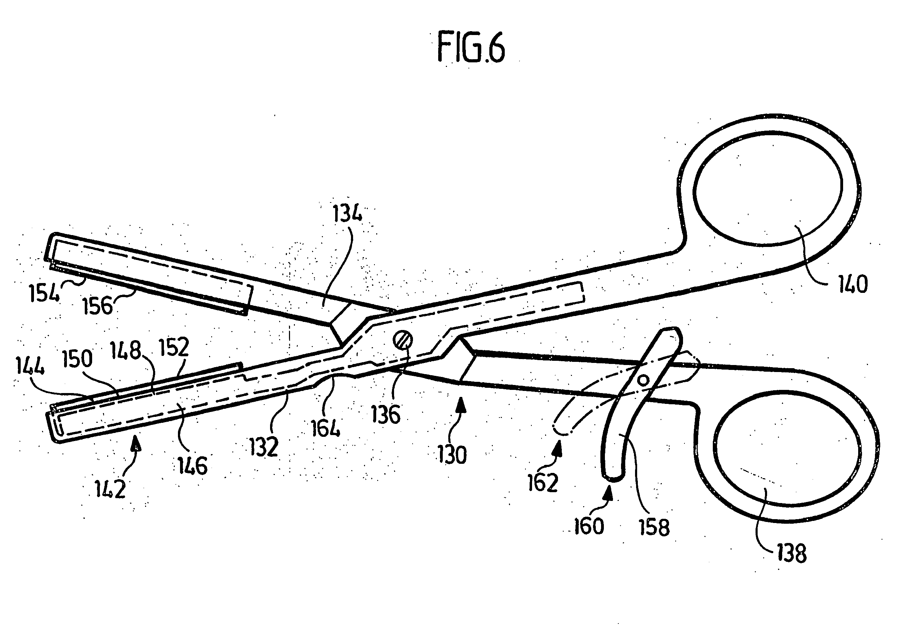

In Figur 6 ist ein zweites Ausführungsbeispiels eines chirurgischen Instruments in der Form einer Schneid-Klemme 130 gezeigt. Diese weist einen ersten Schenkel 132 und einen zweiten 134 auf, welche über ein Drehgelenk 136 gelenkig miteinander verbunden sind. Zur Handhabung weisen dabei die beiden Schenkel 132 und 134 jeweils an ihrem dem Wirkende abgewandten Ende einen Fingerbügel 138 bzw. 140 auf.

Mittels des Drehgelenks 136 ist so ein Klemmenmaul 142 gebildet, dessen Maulbreite durch die relative Drehstellung zwischen dem ersten Schenkel 132 und dem zweiten Schenkel 134 bestimmt ist und in dem sich ein Gegenstand fassen läßt.

An dem ersten Schenkel 132 ist dem zweiten Schenkel 134 im Bereich des Klemmenmauls 142 zugewandt ein Messerlager 144 angeordnet, welches grundsätzlich gleich ausgebildet ist wie das in Figur 2 gezeigte Messerlager 122. Bei dem in Figur 6 gezeigten Ausführungsbeispiel ist dabei ein Messer 146 mit einer Schneidkante 148 unverschieblich an dem ersten Schenkel 132 angeordnet. Mittels eines elastischen Polsters 150 ist eine Anlagefläche 152 entsprechend der Anlagefläche 60 gemäß dem ersten Ausführungsbeispiel gebildet.

Der zweite Schenkel 134 weist ein Gegenlager 154 mit einer Anlagefläche 156 auf, wobei diese wiederum dem ersten Schenkel 132 zugewandt ist.

Das Messerlager 144 funktioniert genauso wie oben anhand des Messerlagers 22 beschrieben, das heißt durch Kraftbeaufschlagung des Polsters 150 verändert sich die relative Lage zwischen der Schneidkante 148 des Messers 146 und der Anlagefläche 152, so daß durch entsprechende Kraftbeaufschlagung steuerbar ist, ob das Messer in einer Nichtwirkstellung (reine Klemmwirkung der Schneid-Klemme 130) oder in einer Wirkstellung (Schneidwirkung der Schneid-Klemme 130) liegt. In der Nichtwirkstellung läßt sich beispielsweise ein Faden fassen und in der Wirkstellung läßt sich dieser dann durchschneiden.

Über die Fingerbügel 138 und 140 werden zum Fassen eines Gegenstands die beiden Schenkel 132 und 134 aufeinander zu bewegt. An beispielsweise dem zweiten Schenkel 134 ist dabei zur Ausbildung einer Begrenzungsvorrichtung ein Begrenzungsbügel 158 schwenkbar angeordnet. Dieser Begrenzungsbügel weist eine erste Stellung 160 auf (in Figur 6 in durchgezogenen Linien), welcher die Öffnungsweite des Klemmenmauls 142 so begrenzt, daß die Schneidkante 148 unterhalb der Anlagefläche 152 liegt, das heißt, daß ein Gegenstand ohne Schneidwirkung gefaßt werden kann. In einer zweiten Stellung 162, welche in Figur 6 in strichpunktierten Linien gezeigt ist, ist dagegen diese Begrenzung aufgehoben, so daß die Schneidkante 148, welche bei dem gezeigten Ausführungsbeispiel im wesentlichen parallel zu einem Schenkelboden des ersten Schenkels 142 orientiert ist, über die Anlagefläche 152 hinaustreten kann.

Es kann vorgesehen sein, daß die erste Stellung 160 selber einstellbar ist, so daß ein Operateur die Dimensionen des zu fassenden Gegenstandes berücksichtigen kann, bevor eine Schneidwirkung eintritt.

Wenn der Begrenzungsbügel 158 in der zweiten Stellung 162 ist und sich die beiden Schenkel 132 und 134 aufeinanderzubewegen, dann werden über den Gegenstand auch Kräfte auf die Schenkel ausgeübt. Bei einer Variante eines bevorzugten Ausführungsbeispiels ist der erste Schenkel 132 dabei so ausgebildet, daß er sich durch elastische Verformung von dem zweiten Schenkel 134 wegbewegen kann. Beispielsweise ist dazu eine Ausnehmung 164 vorgesehen, welche in dem ersten Schenkel 132 an einer dem zweiten Schenkel 134 gegenüberliegenden Seite gebildet ist. Durch diese Ausnehmung 164 ist im Bereich der Ausnehmung die Schenkeldicke vermindert.

Dadurch läßt es sich erreichen, daß die Schneidkante 150 beim Schließen des Klemmenmauls 142 in das Gegenlager 154 mit einem entsprechenden Polster 166 anliegt, um so dazwischen den Gegenstand zuhalten. Bei weiterem Schließen verbiegt sich der erste Schenkel 132 im Bereich des Klemmenmauls 142 nach außen, die Schneidkante 150 tritt über die Anlagefläche 152 hinaus und schneidet in den Gegenstand. Ist dieser ein Faden, so läßt er sich damit durchtrennen.

Das Messer 146 ist dabei als Einlegeteil ausgebildet, welches sich insbesondere durch Sicherung der Drehbarkeit der beiden Schenkel 132 und 134 relativ zueinander und im Bereich des Klemmauls 142 über das Drehgelenk 136 hinaus zu dem Fingerbügel 140 hin erstreckt. Das Messer 146 ist dazu insbesondere in ein Kunststoffmaterial des Schenkels 132 eingebettet.

## Patentansprüche

1. Chirurgisches Instrument mit einem Schenkel (12; 132), welcher an einen Gegenstand anlegbar ist,
wobei an dem Schenkel (12, 132) ein Messerlager (22; 144) mit einem Messer (38; 146) angeordnet ist, das Messerlager (22; 146) eine Anlagefläche (60; 152) für den Gegenstand aufweist und das Messerlager (22; 144) so ausgebildet ist, daß in einer Nichtwirkstellung (58) eine Schneidkante (42; 148) des Messers (38; 146) unterhalb der Anlagefläche (60; 162) liegt und in einer Wirkstellung (60) des Messers (38; 146) die Schneidkante (42; 148) freigegeben ist, **dadurch gekennzeichnet, daß** in dem Messerlager (22; 142) ein Polster (54; 56; 150) aus einem elastischen Material angeordnet ist, welches mindestens in der Nichtwirkstellung (58) die Anlageflächen (60) für den Gegenstand bildet und daß das Polster (54; 56; 150) so zusammendrückbar ist, daß die Schneidkante (42; 148) des Messers (38; 146) freigegeben ist.

2. Chirurgisches Instrument nach Anspruch 1, **gekennzeichnet durch** einen weiteren Schenkel (14; 134) wobei die beiden Schenkel (12, 14; 132; 134) relativ zueinander beweglich sind.

3. Chirurgisches Instrument nach Anspruch 2, **dadurch gekennzeichnet, daß** die Anlagefläche (60; 152) des Messerlagers (22; 144) dem weiteren Schenkel (14; 134) zugewandt angeordnet ist.

4. Chirurgisches Instrument nach Anspruch 2 oder 3, **dadurch gekennzeichnet, daß** der weitere Schenkel (14; 134) ein Gegenlager (68; 154) mit einer Anlagefläche (72; 156) für den Gegenstand aufweist.

5. Chirurgisches Instrument nach einem der vorangehenden Ansprüche, **dadurch gekennzeichnet, daß** der Übergang der Schneidkante (42; 148) von der Nichtwirkstellung (58) in die Wirkstellung (66) durch Kraftbeaufschlagung des Schenkels (12; 132) relativ zum Gegenstand steuerbar ist.

6. Chirurgisches Instrument nach einem der vorangehenden Ansprüche, **dadurch gekennzeichnet, daß** in der Wirkstellung (66) des Messers (38; 146) die Schneidkante (42; 148) oberhalb der Anlagefläche (60; 152) positioniert ist.

7. Chirurgisches Instrument nach einem der vorangehenden Ansprüche, **dadurch gekennzeichnet, daß** die relative Lage zwischen Anlagefläche (60; 152) und Schneidkante (42; 148) steuerbar ist.

8. Chirurgisches Instrument nach Anspruch 7, **dadurch gekennzeichnet, daß** die relative Lage zwischen Anlagefläche (60; 152) und Schneidkante (42; 148) durch Kraftbeaufschlagung des Schenkels (12; 132) steuerbar ist.

9. Chirurgisches Instrument nach einem der vorangehenden Ansprüche, **dadurch gekennzeichnet, daß** das Messer (38) zwischen einem ersten Polsterteil (54) und einem zweiten Polsterteil (56) angeordnet ist.

10. Chirurgisches Instrument nach einem der vorangehenden Ansprüche, **dadurch gekennzeichnet, daß** in einer Wirkstellung (66) des Messers (38) die Anlagefläche zumindest teilweise durch eine Polsteraufnahme des Messerlagers (22) gebildet ist.

11. Chirurgisches Instrument nach einem der Ansprüche 4 bis 10, **dadurch gekennzeichnet, daß** das Gegenlager (68; 154) mit einem Polster (70; 166) aus einem elastischen Material versehen ist.

12. Chirurgisches Instrument nach einem der vorangehenden Ansprüche, **dadurch gekennzeichnet, daß** das Messer (38) zur Durchführung einer Schneidbewegung an dem Schenkel (12) beweglich gelagert ist.

13. Chirurgisches Instrument nach Anspruch 12, **dadurch gekennzeichnet, daß** das Messer (38) in Längsrichtung des Schenkels (12) verschieblich gelagert ist.

14. Chirurgisches Instrument nach Anspruch 12 oder 13, **dadurch gekennzeichnet, daß** das Messer (38) ein Griffelement (50) zu dessen Betätigung aufweist.

15. Chirurgisches Instrument nach einem der Ansprüche 12 bis 15, **dadurch gekennzeichnet, daß** eine Sicherungsvorrichtung (106) gegen eine unbeabsichtigte Schneidbewegung des Messers (38) vorgesehen ist.

16. Chirurgisches Instrument nach einem der vorangehenden Ansprüche, **dadurch gekennzeichnet, daß** die Schneidkante (42) quer zur Anlagefläche (60) orientiert ist.

17. Chirurgisches Instrument nach einem der vorangehenden Ansprüche, **dadurch gekennzeichnet, daß** das Messer (38) ein hakenförmiges Element (40) aufweist, an welchem die Schneidkante (42) sitzt.

18. Chirurgisches Instrument nach einem der Ansprüche 1 bis 15, **dadurch gekennzeichnet, daß** die Schneidkante (148) des Messers (146) im wesentlichen parallel zu der Anlagefläche (152) orientiert ist.

19. Chirurgisches Instrument nach einem der Ansprüche 12 bis 18, **dadurch gekennzeichnet, daß** ein Anschlag zur Begrenzung der Beweglichkeit des Messers vorgesehen ist.

20. Chirurgisches Instrument nach Anspruch 19, **dadurch gekennzeichnet, daß** ein Anschlag an einem vorderen Ende des Schenkels (12) angeordnet ist.

21. Chirurgisches Instrument nach einem der Ansprüche 12 bis 20, **dadurch gekennzeichnet, daß** das Messer (94) und eine Messerführung so ausgebildet sind, daß bei Verschiebung des Messers (94) sich die Lage der Schneidkante (96) relativ zur Anlagefläche (60) ändert.

22. Chirurgisches Instrument nach einem der Ansprüche 2 bis 21, **dadurch gekennzeichnet, daß** eine Begrenzungsvorrichtung (158) vorgesehen ist, mittels welcher die Relativbewegung der beiden Schenkel (132, 134) zueinander derart begrenzbar ist, daß das Messer in der Nichtwirkstellung (58) bleibt.

23. Chirurgisches Instrument nach Anspruch 22, **dadurch gekennzeichnet, daß** durch die Begrenzungsvorrichtung (158) der Anpreßdruck des Schenkels (132) mit dem Messerlager (144) auf einen Gegenstand an der Anlagefläche (152) begrenzbar ist.

24. Chirurgisches Instrument nach Anspruch 22 oder 23, **dadurch gekennzeichnet, daß** die. Begrenzungsvorrichtung (158) mindestens zwischen einer Begrenzungsstellung (160) und einer Nichtbegrenzungsstellung (162) umschaltbar ist.

25. Chirurgisches Instrument nach einem der vorangehenden Ansprüche, **gekennzeichnet durch** eine Ausbildung als Pinzette (10), bei der der Schenkel (12) mit einem weiteren Schenkel (14) derart elastisch verbunden ist, daß die beiden Schenkel (12, 14) relativ zueinander beweglich sind.

26. Chirurgisches Instrument nach einem der Ansprüche 1 bis 24, **gekennzeichnet durch** einen weiteren Schenkel (134), welcher drehbar an dem Schenkel (132) angeordnet ist.

27. Chirurgisches Instrument nach Anspruch 26, **gekennzeichnet durch** eine Ausbildung als Klemme (130).

28. Chirurgisches Instrument nach Anspruch 27, **dadurch gekennzeichnet, daß** der Schenkel (132) mit dem Messerlager (144) so ausgebildet ist, daß bei Schließen der Klemme (130) die Anlagefläche (152) an einer Anlagefläche des anderen Schenkels (134) im wesentlichen anliegt.

29. Chirurgisches Instrument nach Anspruch 27 oder 28, **dadurch gekennzeichnet, daß** der Schenkel (132) so elastisch ausgebildet ist, daß das Messerlager (144) relativ zu einer Längsrichtung des Schenkels (132) beweglich ist.

30. Chirurgisches Instrument nach einem der Ansprüche 27 bis 29, **dadurch gekennzeichnet, daß** der Schenkel (132) so elastisch ausgebildet ist, daß das Messerlager (144) bei Anliegen an dem anderen Schenkel (134) und bei Kraftausübung der beiden Schenkel (132, 134) relativ zueinander von dem anderen Schenkel (134) wegbeweglich ist.

31. Chirurgisches Instrument nach Anspruch 29 oder 30, **dadurch gekennzeichnet, daß** der Schenkel (132) an einer dem anderen Schenkel (134) abgewandten Seite eine Ausnehmung (164) aufweist.

32. Chirurgisches Instrument nach einem der vorangehenden Ansprüche, **dadurch gekennzeichnet, daß** ein Schenkel (12; 132; 134) aus einem Kunststoffmaterial hergestellt ist.

## Claims

1. A surgical instrument having an arm (12; 132) able to be placed against an object, wherein a blade bearing (22; 144) with a blade (38; 146) is arranged on the arm (12, 132), the blade bearing (22; 146) has a contact surface (60; 152) for the object and the blade bearing (22; 144) is such in design that a cutting edge (42; 148) of the blade (38; 146) lies below the contact surface (60; 162) in a non-operational position and the cutting edge (42; 148) is released in an operational position (60) of the blade (38; 146), **characterised in that** a pad (54; 56; 150) of a resilient material is arranged in the blade bearing (22; 142) and forms the contact surfaces (60) for the object at least in the non-operational position (58), and **in that** the pad (54; 56; 150) is compressible such that the cutting edge (42; 148) of the blade (38; 146) is released.

2. A surgical instrument according to claim 1, **characterised by** another arm (14; 134), the two arms (12, 14; 132; 134) being movable relative to one another.

3. A surgical instrument according to claim 2, **characterised in that** the contact surface (60; 152) of the blade bearing (22; 144) is arranged facing the additional arm (14; 134).

4. A surgical instrument according to claim 2 or 3, **characterised in that** the additional arm (14; 134) has a counter bearing (68; 154) with a contact surface (72; 156) for the object.

5. A surgical instrument according to any one of the preceding claims, **characterised in that** the change over of the cutting edge (42; 148) from the non-operational position (58) into the operational position (66) can be controlled by the arm (12; 132) being acted upon by force relative to the object.

6. A surgical instrument according to any one of the preceding claims, **characterised in that** the cutting edge (42; 148) is positioned above the contact surface (60; 152) in the operational position (66) of the blade (38; 146).

7. A surgical instrument according to any one of the preceding claims, **characterised in that** the relative position between the contact surface (60; 152) and the cutting edge (42; 148) can be controlled.

8. A surgical instrument according to claim 7, **characterised in that** the relative position between the contact surface (60; 152) and the cutting edge (42; 148) can be controlled by the action of force upon the arm (12; 132).

9. A surgical instrument according to any one of the preceding claims, **characterised in that** the blade (38) is arranged between a first pad part (54) and a second pad part (56).

10. A surgical instrument according to any one of the preceding claims, **characterised in that** in an operational position (66) of the blade (38), the contact surface is formed at least partially by a pad receiver of the blade bearing (22).

11. A surgical instrument according to any one of claims 4 to 10, **characterised in that** the counter bearing (68; 154) is provided with a pad (70; 166) of a resilient material.

12. A surgical instrument according to any one of the preceding claims, **characterised in that** the blade (38) is movably mounted on the arm (12) in order to carry out a cutting movement.

13. A surgical instrument according to claim 12, **characterised in that** the blade (38) is mounted so as to be displaceable in the longitudinal direction of the arm (12).

14. A surgical instrument according to claim 12 or 13, **characterised in that** the blade (38) has a grip element (50) for actuation thereof.

15. A surgical instrument according to any one of claims 12 to 15, **characterised in that** a securing device (106) to prevent an unintentional cutting movement of the blade (38) is provided.

16. A surgical instrument according to any one of the preceding claims, **characterised in that** the cutting edge (42) is oriented transversely to the contact surface (60).

17. A surgical instrument according to any one of the preceding claims, **characterised in that** the blade (38) has a hook-shaped element (40) on which there is the cutting edge (42).

18. A surgical instrument according to any one of claims 1 to 15, **characterised in that** the cutting edge (148) of the blade (146) is oriented substantially parallel to the contact surface (152).

19. A surgical instrument according to any one of claims 12 to 18, **characterised in that** a stop to limit movability of the blade is provided.

20. A surgical instrument according to claim 19, **characterised in that** a stop is arranged on a front end of the arm (12).

21. A surgical instrument according to any one of claims 12 to 20, **characterised in that** the blade (94) and a blade guide are designed such that the position of the cutting edge (96) relative to the contact surface (60) alters upon displacement of the blade (94).

22. A surgical instrument according to any one of claims 2 to 21, **characterised in that** a limiting device (158) is provided, by means of which the relative motion of the two arms (132, 134) in relation to one another can be limited in such a manner that the blade remains in the non-operational position (58).

23. A surgical instrument according to claim 22, **characterised in that** the contact pressure of the arm (132) with the blade bearing (144) on an object can be limited at the contact surface (152) by the limiting device (158).

24. A surgical instrument according to claim 22 or 23, **characterised in that** the limiting device (158) can be changed over at least between a limiting position (160) and a non-limiting position (162).

25. A surgical instrument according to any one of the preceding claims, **characterised by** a design as forceps (10) with which the arm (12) is resiliently connected to an additional arm (14) in such a manner that the two arms (12, 14) are movable relative to one another.

26. A surgical instrument according to any one of claims 1 to 24, **characterised by** an additional arm (134) rotatably arranged on the arm (132).

27. A surgical instrument according to claim 26, **characterised by** a design as a clamp (130).

28. A surgical instrument according to claim 27, **characterised in that** the arm (132) with the blade bearing (144) is designed in such a manner that the contact surface (152) substantially rests against a contact surface of the other arm (134) upon closure of the clamp (130).

29. A surgical instrument according to claim 27 or 28, **characterised in that** the arm (132) is resilient such that the blade bearing (144) is movable relative to a longitudinal direction of the arm (132).

30. A surgical instrument according to any one of claims 27 to 29, **characterised in that** the arm (132) is resilient such that the blade bearing (144) can be moved away from the other arm (134) upon contact with the other arm (134) and when the two arms (132, 134) exert force relative to one another.

31. A surgical instrument according to claim 29 or 30, **characterised in that** the arm (132) has a recess (164) at a side remote from the other arm (134).

32. A surgical instrument according to any one of the preceding claims, **characterised in that** an arm (12; 132; 134) is manufactured of a plastics material.

## Revendications

1. Instrument chirurgical comprenant une branche (12; 132) qui peut être appliquée contre un objet,
dans lequel sur la branche (12, 132) est agencée une monture de couteau (22 ; 144) avec un couteau (38 ; 146), la monture de couteau (22 ; 146) présente une surface d'appui (60 ; 152) pour ledit objet, et la monture de couteau (22 ; 144) est conçue de façon à ce que dans une position non active (58), un tranchant de coupe (42 ; 148) du couteau (38 ; 146) se situe en-dessous de la surface d'appui (60 ; 162), et dans une position active (60) du couteau (38 ; 146), le tranchant de coupe (42 ; 148) soit dégagé,
**caractérisé en ce que** dans la monture de couteau (22 ; 142) est agencé un rembourrage (54 ; 56 ; 150) en un matériau élastique, qui, au moins dans la position non active (58), forme les surfaces d'appui (60) pour l'objet, et **en ce que** le rembourrage (54 ; 56 ; 150) peut être compressé de manière telle que le tranchant de coupe (42 ; 148) du couteau (38 ; 146) soit dégagé.

2. Instrument chirurgical selon la revendication 1, **caractérisé par** une autre branche (14 ; 134), les deux branches (12, 14 ; 132, 134) étant mobiles relativement l'une par rapport à l'autre.

3. Instrument chirurgical selon la revendication 2, **caractérisé en ce que** la surface d'appui (60 ; 152) de la monture de couteau (22 ; 144) est agencée de manière à être dirigée vers ladite autre branche (14 ; 134).

4. Instrument chirurgical selon la revendication 2 ou la revendication 3, **caractérisé en ce que** ladite autre branche (14 ; 134) présente un appui de butée conjugué (68 ; 154) comportant une surface d'appui (72 ; 156) pour l'objet.

5. Instrument chirurgical selon l'une des revendications précédentes, **caractérisé en ce que** le passage du tranchant de coupe (42 ; 148) de la position non active (58) à la position active (66) peut être commandé par une sollicitation mécanique de la branche (12 ; 132) relativement à l'objet.

6. Instrument chirurgical selon l'une des revendications précédentes, **caractérisé en ce que** dans la position active (66) du couteau (38 ; 146), le tranchant de coupe (42 ; 148) est positionné au-dessus de la surface d'appui (60 ; 152).

7. Instrument chirurgical selon l'une des revendications précédentes, **caractérisé en ce qu'**il est possible de commander la position relative entre la surface d'appui (60 ; 152) et le tranchant de coupe (42 ; 148).

8. Instrument chirurgical selon la revendication 7, **caractérisé en ce qu'**il est possible de commander la position relative entre la surface d'appui (60 ; 152) et le tranchant de coupe (42 ; 148) par sollicitation mécanique de la branche (12 ; 132).

9. Instrument chirurgical selon l'une des revendications précédentes, **caractérisé en ce que** le couteau (38) est agencé entre une première pièce de rembourrage (54) et une deuxième pièce de rembourrage (56).

10. Instrument chirurgical selon l'une des revendications précédentes, **caractérisé en ce que** dans une position active (66) du couteau (38), la surface d'appui est formée au moins en partie par un logement de réception de rembourrage de la monture de couteau (22).

11. Instrument chirurgical selon l'une des revendications 4 à 10, **caractérisé en ce que** l'appui de butée conjugué (68 ; 154) est pourvu d'un rembourrage (70 ; 166) en un matériau élastique.

12. Instrument chirurgical selon l'une des revendications précédentes, **caractérisé en ce que** le couteau (38) est monté mobile sur la branche (12) en vue d'effectuer un mouvement de coupe.

13. Instrument chirurgical selon la revendication 12,
**caractérisé en ce que** le couteau (38) est monté de manière à pouvoir coulisser dans la direction longitudinale de la branche (12).

14. Instrument chirurgical selon la revendication 12 ou la revendication 13, **caractérisé en ce que** le couteau (38) présente un élément de préhension (50) pour son actionnement.

15. Instrument chirurgical selon l'une des revendications 12 à 14, **caractérisé en ce qu'**il est prévu un dispositif de sécurité (106) à l'encontre d'un mouvement de coupe involontaire du couteau (38).

16. Instrument chirurgical selon l'une des revendications précédentes, **caractérisé en ce que** le tranchant de coupe (42) est orienté transversalement à la surface d'appui (60).

17. Instrument chirurgical selon l'une des revendications précédentes, **caractérisé en ce que** le couteau (38) présente un élément en forme de crochet (40) sur lequel est placé le tranchant de coupe (42).

18. Instrument chirurgical selon l'une des revendications 1 à 15, **caractérisé en ce que** le tranchant de coupe (148) du couteau (146) est orienté sensiblement de manière parallèle à la surface d'appui (152).

19. Instrument chirurgical selon l'une des revendications 12 à 18, **caractérisé en ce qu'**il est prévu une butée pour limiter la mobilité du couteau.

20. Instrument chirurgical selon la revendication 19, **caractérisé en ce qu'**une butée est agencée à une extrémité avant de la branche (12).

21. Instrument chirurgical selon l'une des revendications 12 à 20, **caractérisé en ce que** le couteau (94) et un guidage de couteau sont conçus de façon telle que, lors du coulissement du couteau (94), la position du tranchant de coupe (96) par rapport à la surface d'appui (60) varie.

22. Instrument chirurgical selon l'une des revendications 2 à 21, **caractérisé en ce qu'**il est prévu un dispositif de limitation (158) au moyen duquel il est possible de limiter le mouvement relatif des deux branches (132, 134) l'une vers l'autre, de façon telle que le couteau reste dans la position non active (58).

23. Instrument chirurgical selon la revendication 22, **caractérisé en ce que** grâce au dispositif de limitation (158), il est possible de limiter la pression d'appui de la branche (132) avec la monture de couteau (144), sur un objet, au niveau de la surface d'appui (152).

24. Instrument chirurgical selon la revendication 22 ou la revendication 23, **caractérisé en ce que** le dispositif de limitation (158) peut être commuté au moins entre une position de limitation (160) et une position de non limitation (162).

25. Instrument chirurgical selon l'une des revendications précédentes, **caractérisé par** une réalisation en tant que pincette (10) dans laquelle la branche (12) est reliée de manière élastique avec une autre branche (14), de façon telle que les deux branches (12, 14) soient mobiles relativement l'une par rapport à l'autre.

26. Instrument chirurgical selon l'une des revendications 1 à 24, **caractérisé par** une autre branche (134) qui est agencée de manière rotative sur la branche (132).

27. Instrument chirurgical selon la revendication 26, **caractérisé par** une réalisation en tant que pince (130).

28. Instrument chirurgical selon la revendication 27, **caractérisé en ce que** la branche (132) avec la monture de couteau (144) est conçue de façon telle que lors de la fermeture de la pince (130), la surface d'appui (152) s'appuie sensiblement sur une surface d'appui de l'autre branche (134).

29. Instrument chirurgical selon la revendication 27 ou la revendication 28, **caractérisé en ce que** la branche (132) est d'une configuration élastique telle, que la monture de couteau (144) soit mobile par rapport à une direction longitudinale de la branche (132).

30. Instrument chirurgical selon l'une des revendications 27 à 29, **caractérisé en ce que** la branche (132) est d'une configuration élastique telle, que la monture de couteau (144), lors de son appui contre l'autre branche (134) et lorsque les deux branches (132, 134) exercent une force relativement l'une à l'encontre de l'autre, puisse être éloignée de l'autre branche (134).

31. Instrument chirurgical selon la revendication 29 ou la revendication 30, **caractérisé en ce que** la branche (132) présente un évidement (164) sur un côté opposé à celui dirigé vers l'autre branche (134).

32. Instrument chirurgical selon l'une des revendications précédentes, **caractérisé en ce qu'**une branche (12 ; 132 ; 134) est fabriquée en un matériau du type matière plastique.
